# EUROPÄISCHE PATENTANMELDUNG

(11) **EP 1 048 269 A1**
(43) Veröffentlichungstag der Anmeldung: **02.11.2000**
(21) Anmeldenummer: 00201517.0
(22) Anmeldetag: 20.04.2000
(51) Int. Cl.: A61B 6/06, G03B 42/04

(54) **Röntgeneinrichtung mit Detektion von Blendenplattenkanten**

(30) Priorität: 30.04.1999 DE 19919849
(71) Anmelder: Philips Corporate Intellectual Property GmbH, 52064 Aachen (DE); Koninklijke Philips Electronics N.V., 5621 BA Eindhoven (NL)
(72) Erfinder: Kuhn, Michael Harald, 52064 Aachen (DE); Buzug, Thorsten, 52064 Aachen (DE); Wiemker, Rafael, 52064 Aachen (DE)
(74) Vertreter: Volmer, Georg, Dipl.-Ing.

(57) **Zusammenfassung**

In einer Röntgeneinrichtung, die wenigstens eine Strahlungsquelle (1), eine Blendenplatten (2) enthaltende Blendenvorrichtung (8) und eine Aufnahme (3)- und Bildverarbeitungseinheit (4) umfaßt, weisen die Blendenplatten (2) Loch und/oder Kantenmuster (11-14) auf, die nicht anatomische Muster in der Strahlungsaufnahme abbilden, mit denen in der Bildverarbeitungseinheit (4) die Blendenplattenkanten (15) in der Strahlungsaufnahme derektiert werden und damit nur noch der direkt belichtete Bereich des Patienten dargestellt wird.

## Beschreibung

Die Erfindung bezieht sich auf eine Röntgeneinrichtung, die wenigstens eine Strahlungsquelle, eine Blendenplatten enthaltende Blendenvorrichtung und eine Aufnahme- und Bildverarbeitungseinheit umfaßt.

Bei der Untersuchung von Patienten mit Röntgenstrahlung ist die Röntgendosis, mit der der Patient beaufschlagt wird, so gering wie möglich zu halten. Dazu wird der Strahlengang von einer Blendenanordnung begrenzt. Zur Beurteilung eines Röntgenbildes ist eine unverfälschte Darstellung selbst der kleinsten Graustufenunterschiede notwendig.

In der DE 195 39 602 A1 wird ein Röntgenuntersuchungsgerät mit zwei verstellbaren Blendenanordnungen beschrieben. Eine erste Blendenanordnung begrenzt ein von einem Röntgenstrahler erzeugtes Röntgenstrahlenbündel, welches einen Untersuchungsbereich durchsetzt und danach eine zweite Blendenanordnung passiert, bevor es auf eine Einheit zum Aufzeichnen der Röntgenaufnahme trifft. Die zweite Blendenanordnung ist dabei so ausgestaltet, daß in der Röntgenaufnahme erkennbar ist, ob das von der ersten Blendenanordnung ausgeblendete Röntgenstrahlenbündel wesentlich größer als das von der zweiten Blendenanordnung ausgeblendete Röntgenstrahlenbündel ist. Dazu weist die zweite Blendenanordnung in ihren Blendenplatten zu deren Kanten senkrechte Einschnitte die am Rand der Röntgenaufnahme streifenförmige Ausbuchtungen abbilden Mit dieser Anordnung wird erreicht, daß die Röntgenaufnahme eine schaffe Begrenzung aufweist, da durch Begrenzung mit nur der ersten Blendenanordnung unscharfe Kanten auf der Röntgenaufnahme entstehen. Mit der zweiten Blendenanordnung, die mir geringem Abstand vor der Einheit zur Aufnahme der Röntgenaufnahme angeordnet ist, wird daß die Kanten der Blendenplatten auf der Röntgenaufnahme schaff abgebildet werden.

In der DE 196 37 918 A1 wird eine medizinische Röntgeneinrichtung beschrieben, bei der Mittel zur Erzeugung einer einrichtungsspezifischen und/oder komponentenspezifischen Markierung oder Datenkennung automatisch einer zu erzeugenden Aufnahme zugeordnet werden. Hierbei handelt es sich um Eintiefungen, Markierungen oder Durchbrüche die so auf der Aufnahme abgebildet werden, daß die Aufnahmen bei späterer Betrachtung durch den Arzt dadurch identifiziert werden können, oder dem Arzt mittels der Markierungen Zusatzinformationen für die Aufnahme übermittelt werden.

Durch das Begrenzen des Strahlengangs mit der Blendenanordnung entstehen auf der Strahlungsaufnahme Kanten, die den mit Röntgenstrahlen durchsetzen Bereich vom nicht durchsetzten Bereich trennen. Die gesamte Strahlungsaufnahme weist viele unterschiedliche Schwan-Weiß bzw. Grauwertübergänge auf, die einerseits durch Grauwertübergänge beispielsweise an Knochen, andererseits aber auch durch die Kanten der Blendenplatten erzeugt werden. Der Arzt benötigt für die Beurteilung der Strahlungsaufnahme jedoch nur den belichteten Bereich. Bei der Beurteilung der gesamten Strahlungsaufnahme kann es zur Fehlinterpretation kommen, da auch im nicht belichteten Bereich durch Streuung verursachte unterschiedliche Graustufen abgebildet werden.

Aufgabe der Erfindung ist es, die Kanten der Blendenplatten zu detektieren, um zur Betrachtung der Strahlungsaufnahme nur den exponierten Bereich darzustellen.

Diese Aufgabe wird dadurch gelöst, daß die Blendenplatten Loch und/oder Kantenmuster aufweisen, die nicht anatomische Muster in der Strahlungsaufnahme abbilden und daß die Bildverarbeitungseinheit zur Detektion der Blendenplattenkanten in der Strahlungsaufnahme vorgesehen ist.

Die Blendenplatten weisen Loch und/oder Kantenmuster auf, die in der Anatomie des Menschen nicht vorkommen. Die Blendenplatten werden so in den Strahlengang der Strahlungsquelle gebracht, daß der von den Strahlen durchsetzte Bereich des Patienten von außen beschränkt wird. Dazu sind die Blendenplatten in der Blendenvorrichtung bewegbar angeordnet. Die Bewegung der Blendenplatten kann manuell oder auch automatisch mittels Stellmotoren erfolgen, die von einer Steuerung angetrieben werden. Die Lochmuster sind in einem bekannten Abstand vom Rand der Blendenplatten angebracht. Die aus mehreren Löchern bestehenden Lochmuster sind für Strahlung durchlässig und werden dadurch auf der Strahlungsaufnahme abgebildet. Die Kantenmuster sind an den Kanten der Blendenplatten angeordnet und weisen ebenfalls eine nicht anatomische Form auf, die dann auf der Strahlungsaufnahme abgebildet wird. Nachdem die Strahlung die Blendenplatten passiert hat, durchsetzt sie den zu untersuchenden Bereich des Patienten. Dort wird die Strahlung vom Gewebe und den Knochen des Patienten beeinflußt bzw. absorbiert und trifft dann auf die Aufnahmeeinheit, in der dann eine Strahlungsaufnahme abgebildet wird, die einerseits die Loch- und/oder Kantenmuster und andererseits unterschiedliche Graustufen enthält, da durch die unterschiedliche Dichte von Gewebe und Knochen die Strahlung unterschiedlich stark absorbiert wird.

Diese Strahlungsaufnahme wird der Bildverarbeitungseinheit zugeführt. Hier wird die Strahlungsaufnahme nach den bekannten Loch und/oder Kantenmustern abgesucht, die im folgenden nur noch als Muster bezeichnet werden. Da die Muster keine anatomische Form aufweisen, kann bei der Kantendetektion eine Verwechslung mit beispielsweise Übergängen von Knochen zu weichem Gewebe ausgeschlossen werden. Nachdem die bekannten Muster gefunden wurden, wird anhand der bekannten Anordnung der Muster in den Blendenplatten die Position der Kanten in der Strahlungsaufnahme berechnet. Die Kanten der Blendenplatten bilden auf der Strahlungsaufnahme einen relativ starken Grauwertübergang ab, so daß dieser dann mittels dieser Berechnung eindeutig identifiziert werden kann. Nach der Detektion der Kanten der Blendenplatten werden die Bereiche der Strahlungsaufnahme außerhalb dieser Kanten unterdrückt und dem Betrachter nicht angezeigt. Dem Betrachter wird auf einer Anzeigeeinheit nur der von der Strahlung durchsetzte Bereich des Patienten dargestellt, so daß dieser nicht durch Streuung der Strahlung verursachte Grauwertübergänge in seine Diagnosestellung einbezieht. Dadurch wird die Strahlendosis, der der Patient ausgesetzt wird, auf ein notwendiges Minimum reduziert.

In einer vorteilhaften Ausgestaltung der Erfindung ist das Lochmuster als Perforation realisiert. Dabei sind die Lochmuster in mehreren Reihen parallel zur Kante der Blendenplatte angeordnet. Die Lochmuster weisen in jeder Reihe eine unterschiedliche Anordnung auf. Die Abstände zwischen den einzelnen Löchern sowie die Durchmesser und Form der Löcher können variiert werden. Durch die Verwendung von unterschiedlichen Arten von Perforationen kann das auf der Strahlungsaufnahme abgebildete Muster an den jeweiligen Einsatzzweck angepaßt werden. So daß bei Auf-nahmen bestimmter Objekte eine Perforation gewählt wird, deren Abbildung in der Strahlungsaufnahme eindeutig von dem abgebildeten Objekt unterschieden wird und bei der Kantendetektion Verwechslungen ausgeschlossen werden.

Bei einer bevorzugten Ausführung der Erfindung ist vorgesehen, in den Perforationen Informationen zu kodieren. Bei Abbildung mehrerer paralleler Perforationsreihen auf der Strahlungsaufnahme wird mit einem beispielsweise größer werdendem Anstand der Löcher in den Perforationsreihen die Größe des abgedeckten Bereichs in der Strahlungsaufnahme kodiert. Bei der Detektion der Kanten in der Bildverarbeitungseinheit wird damit eine weirere Information mitgeliefert, wieviel Fläche von der maximal möglichen Strahlungsaufnahmefläche durch die Blendenplatten abgedeckt ist.

Ausführungsbeispiele der Erfindung werden im folgenden anhand der Zeichnungen näher erläutert. Es zeigen.
- Figur 1: schematische Darstellung der erfindungsgemäßen Röntgeneinrichtung
- Figur 2 und 3: Blendenplatten
- Figur 4: Strahlungsaufnahme mit nicht anatomischen Mustern

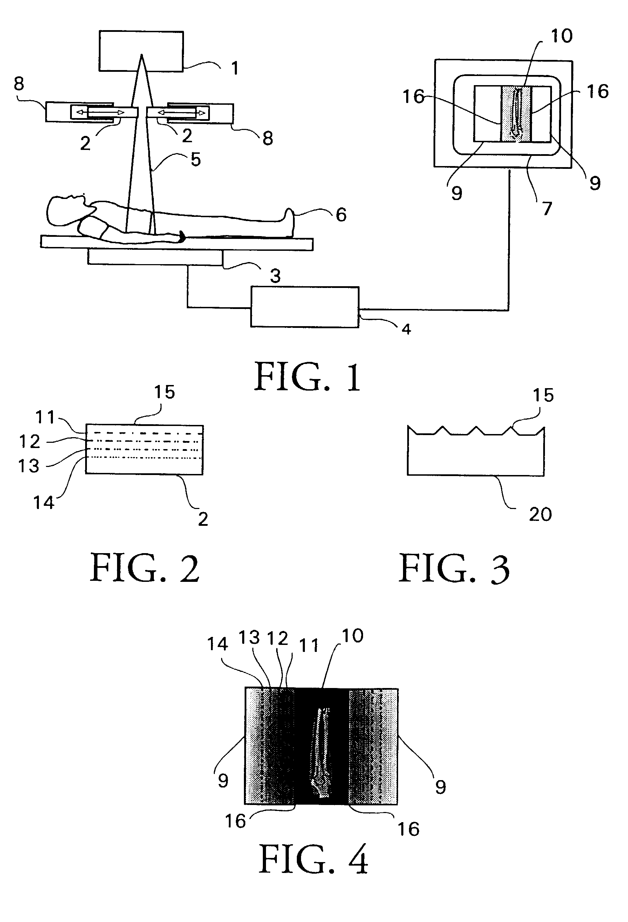

In Figur 1 ist die Röntgeneinrichtung mit einer Röntgenstrahlung erzeugenden Strahlungsquelle 1 dargestellt. Die von der Strahlungsquelle ausgesendete Strahlung trifft auf die Blendenplatten 2 die in einer Blendenvorrichtung 8 verschiebbar gelagert sind. Diese Blendenplatten 2 weisen nicht anatomische, eindeutig erkennbare Perforationen und/oder Kantenmuster auf, die in Figur 1 nicht darstellbar sind.

Durch die Blendenplatten 2 wird der Strahlengang 5 begrenzt und trifft auf den Bereich des Patienten 6 der mittels Röntgenstrahlung untersucht werden soll. Die Röntgenstrahlung durchsetzt den Patienten 6 und wird durch unterschiedliche Gewebedichte und Knochen unterschiedlich stark absorbiert. Mit der dadurch entstehenden unterschiedlichen Strahlungsintensität trifft die Röntgenstrahlung auf die Aufnahmeeinheit 3. In dieser Aufnahmeeinheit wird die Strahlungsaufnahme aufgezeichnet und an die Bildverarbeitungseinheit 4 weitergeleitet. In der Bildverarbeitungseinheit 4 wird die Strahlungsaufnahme nach Loch und/oder Kantenmustern abgesucht, die in den Blendenplatten 2 angeordnet sind und für Röntgenstrahlung durchlässig sind und deshalb in der Strahlungsaufnahme abgebildet werden. Nachdem diese Muster gefunden wurden, wird anhand der bekannten Anordnung der Loch und/ Kantenmuster in den Blendenplatten 2 die Position 16 der Kanten 15 der Blendenplatten 2 identifiziert. Diese Kanten 15 werden in der Strahlungsaufnahme mit einem großen Grauwerteunterschied dargestellt, wie er auch am Übergang von Knochen zu weichem Gewebe auftreten kann. Mithilfe der Loch und/oder Kantenmuster läßt sich die Position 16 der Kanten 15 eindeutig bestimmen. Auf einer Anzeigeeinheit 7 wird nur noch der belichtete Bereich 10 des Patienten mit dem beispielsweise zu untersuchenden Knochen dargestellt. Der von den Blendenplatten 2 abgedeckte Bereich 9 wird nicht dargestellt.

In Figur 2 und 3 sind zwei Blendenplatten 2 und 20 dargestellt, die zur Begrenzung des Strahlengangs 5 eingesetzt werden.

Die Blendenplatte 2 weist Perforationen auf die für Röntgenstrahlung durchlässig sind. Jede der Perforationsreihen 11-14 hat einen unterschiedlichen, charakteristischen Lochabstand. In diesen unterschiedlichen Lochabständen sind Informationen über die Größe des abzudeckenden Bereichs kodiert. In Perforationsreihe 11 ist nur ein kreisförmiges Loch in einer Folge von Langlöchern angeordnet. Diese Perforationsreihe 11 ist mit einem einfachen Abstand von der Blendenkante 15 entfernt. Die Perforationsreihe 12 weist zwei Löcher und ein Langloch auf und hat einen zweifachen Abstand von der Blendenkante 15. Perforationsreihe 13 hat drei Löcher und hat einen dreifachen Abstand und Perforationsreihe 14 hat vier Löcher in Folge und hat einen vierfachen Abstand von der Blendenkante 15.

Die Blendenplatte 20 in Figur 3 weist eine Kante 15 auf, die in der Strahlungsaufnahme besonders einfach wiederzuerkennen ist. Diese Blendenplatte 20 ist mit gleichmäßig beabstandeten Einkerbungen versehen. Diese Kerben werden durch einen Standard Algorithmus zur Kantendetektion zumindest als Unterbrechung in einem langen, geraden Kantenstück detektiert. Dadurch ist die Unterscheidbarkeit von anatomisch bedingten Kanten in der Strahlungsaufnahme gesichert. Eine zusätzliche Verifikation der erkannten Blendenplattenkanten kann anhand der bekannten Abbildungsgeometrie der Röntgeneinrichtung erfolgen, indem der Abstand und /oder Position der Kerben in der Strahlungsaufnahme vorherberechnet und mit dem detektierten Ergebnis in der Strahlungsaufnahme verglichen wird. Die Kante 15 kann durch eine mathematische Funktion beschrieben werden.

In Figur 4 ist eine Strahlungsaufnahme dargestellt, bei der die Randbereiche 9 mittels Blendenplatten 2 abgedeckt sind. Da hier die Perforationsreihen 11-14 sichtbar sind, bedeutet dies, daß nur noch ein minimaler Bereich mit der Röntgenstrahlung belichtet wurde. Die Blendenplatten 2 wurden in der Blendenvorrichtung 8 sehr weit in den Strahlengang 5 hineingeschoben. Die Perforationsreihen 11-14 haben einen bekannten Abstand zur jeweiligen Kante 15 der Blendenplatten 2, mit dem die Kanten 15 in der Strahlungsaufnahme detektiert werden.

In der Bildverarbeitungseinheit 4 wird die Strahlungsaufnahme mit einem Mustererkennungsalgorithmus nach den Abbildungen der Mustern abgesucht, die in die Blendenplatten 2 eingearbeitet sind. Die Strahlungsaufnahme wird typischerweise mit einem digitalen Detektor aufgenommen. Die für die einzelnen Bildpunkte repräsentativen Werte werden zur Bildverarbeitungseinheit 4 übertragen. Die in digitaler Form vorliegende Strahlungsaufnahme wird nach einer Folge von Grauwerten abgesucht, die mit den Perforationsreihen 11-14 der Blendenplatten 2 korrelieren. Wenn diese Folgen von Grauwerten identifiziert sind, wird mittels des bekannten Abstandes der jeweiligen Perforationsreihe 11-14 zur Kante 15 der Blendenplatte 2 die Position 16 der Kante 15 der Blendenplatte 2 in der Strahlungsaufnahme detektiert. Die mit den Blendenplatten 2 abgedeckten Bereiche 9 der Strahlungsaufnahme werden nicht auf der Anzeigeeinheit 7 dargestellt.

Dadurch wird der Arzt bei der Beurteilung der Röntgenaufnahme des Patienten 6 nicht durch Streuung verursachte Grauwerte außerhalb des belichteten Bereichs abgelenkt.

Beim Röntgen von künstlichen Gelenken oder Prothesen sind die Loch- oder Kantenmuster so zu wählen, daß sich die künstlichen Formen eindeutig von den Loch oder Kantenmuster unterscheiden.

## Patentansprüche

1. Röntgeneinrichtung, die wenigstens eine Strahlungsquelle (1), eine Blendenplatten (2) enthaltende Blendenvorrichtung (8) und eine Aufnahme (3)- und Bildverarbeitungseinheit (4) umfaßt,
dadurch gekennzeichnet,
daß die Blendenplatten (2) Loch und/oder Kantenmuster (11-14) aufweisen, die nicht anatomische Muster in einer Strahlungsaufnahme abbilden und
daß die Bildverarbeitungseinheit (4) zur Detektion der Blendenplattenkanten (15) in der Strahlungsaufnahme vorgesehen ist.

2. Röntgeneinrichtung nach Anspruch 1
dadurch gekennzeichnet,
daß die Lochmuster als Perforationen (11-14) realisiert sind, die in Reihen parallel zur Kante (15) der Blendenplatte (2) angeordnet sind und jede Perforationsreihe (11-14) Reihe unterschiedliche Abstände von Löchern aufweist.

3. Röntgeneinrichtung nach Anspruch 1 und 2
dadurch gekennzeichnet,
daß in den Perforationsreihen (11-14) Informationen kodiert sind.

4. Röntgeneinrichtung nach Anspruch 3,
dadurch gekennzeichnet,
daß mit dem Abstand der Löcher in den Perforationsreihen (11-14) die Größe des in der Strahlungsaufnahme von den Blendenplatten (2) abgedeckten Bereichs codiert ist.

5. Blendenplatten einer Blendenvorrichtung,
dadurch gekennzeichnet,
daß die Blendenplatten (2) nicht anatomische Loch- und oder Kantenmuster aufweisen.
